# DEMANDE DE BREVET EUROPEEN

(11) **EP 0 755 943 A1**
(43) Date de publication de la demande: **29.01.1997**
(21) Numéro de dépôt: 96401661.2
(22) Date de dépôt: 25.07.1996
(51) Int. Cl.: C07K 14/18, A61K 39/29, G01N 33/576, C12N 15/51

(54) **Peptide capable d'être reconnu par des anticorps reconnaissant l'antigène C33 du virus de l'hépatite C**

(30) Priorité: 25.07.1995 FR 9509005
(71) Demandeur: BIO MERIEUX, F-69280 Marcy l'Etoile (FR)
(72) Inventeur: Jolivet-Reynaud, Colette, 69500 Bron (FR)
(74) Mandataire: Tonnellier, Jean-Claude

(57) **Abrégé**

Polypeptide capable de réagir spécifiquement avec un anticorps dirigé contre l'antigène C33 du virus de l'hépatite C, caractérisé en ce que sa séquence peptidique comprend tout ou partie de la séquence Asp-Gly-Ala-Lys-Phe-Ser-Ser-Arg-Leu-Gly-Ala-Ala-Gly-Ala, ou une séquence dérivée de ladite séquence peptidique, ladite séquence dérivée étant reconnue par ledit anticorps.

Application notamment à la détection d'anticorps anti-C33.

## Description

La présente invention concerne un polypeptide, capable de se substituer à l'antigène C33 du virus de l'hépatite C (VHC), un polynucléotide dont l'expression correspond audit polypeptide, et leur utilisation à des fins diagnostiques et thérapeutiques.

Le virus de l'hépatite C (VHC) est un virus à ARN simple brin de 9.4 Kb reconnu comme agent responsable de certaines hépatites non-A non-B. Le génome viral de VHC code pour une polyprotéine d'environ 3010 acides aminés qui, après traduction, subit plusieurs étapes de maturation permettant la production d'une part de protéines dites structurales et d'autres part de protéines dites non-structurales (NS). Les protéines struc-turales du virus VHC sont la protéine de la nucléocapside (correspondant à la région C du génome de VHC), la protéine de la matrice (M) et deux peptides d'enveloppe, glycosylés, gp33 (E1) et gp72 (E2/NS1), Les protéines non-structurales sont NS2 qui est une protéine liée à la membrane virale, NS3 qui joue les rôles de protéase et d'hélicase et NS5 qui correspond à la polymérase virale. La fonction de NS4 est encore inconnue. Des études cliniques ont montré que des anticorps spécifiquement dirigés contre les régions conservées de la nucléocapside et contre NS3 apparaissent précocement après l'infection à VHC. Ces deux protéines constituent donc de bons marqueurs pour la réalisation de tests diagnostiques des infections à VHC. D'autres études ont par ailleurs mis en évidence l'existence de plusieurs antigènes immunodominants, notamment l'antigène C33 de la région NS3, et plus particulièrement l'antigène noté C33c correspondant à une fraction de C33 (voir WO 9115771). Toutefois, l'obtention de ces peptides, 266 et 93 acides aminés respectivement, sous une forme isolée, par recombinaison génétique ou par synthèse chimique, présente un certain nombre d'inconvénients liés notamment aux modifications post-traductionnelles nécessaires pour obtenir des peptides suffisamment immunoréactifs, ou aux difficultés de synthèse chimique desdits peptides, en particulier en raison de leur taille.

De façon surprenante, on a découvert un polypeptide synthétisé par voie chimique ou par le biais des méthodologies de recombinaison génétique, et n'ayant subi aucune modification après sa synthèse, qui est capable d'être reconnu par des anticorps spécifiques des infections à VHC, notés anti-VHC, alors que sa séquence n'a pas d'analogie avec celle de la protéine C33.

Avant d'aborder les objets de l'invention, différents termes employés dans la description qui va suivre sont définis ci-après.

Par "polypeptide", on désigne un peptide ou une protéine, consistant en un assemblage de résidus d'acides aminés liés par des liaisons peptidiques, et obtenu par synthèse chimique ou par des techniques de recombinaison génétique. En décrivant les peptides, on emploie souvent l'expression "acide aminé" pour désigner un résidu d'acide aminé présent dans le peptide. Les polypeptides selon l'invention peuvent être obtenus par des méthodes de synthèse classique, par exemple dans un synthétiseur automatique de peptides, ou par les techniques de génie génétique comprenant l'insertion d'une séquence d'ADN codant ledit polypeptide dans un vecteur d'expression tel qu'un plasmide ou un virus comme décrit dans la présente demande, et la transformation de cellules avec ce vecteur d'expression et culture de ces cellules eucaryotes ou procaryotes.

Par "séquence dérivée d'une autre séquence d'acides aminés", on entend une séquence d'acides aminés modifiée par modification chimique d'un ou plusieurs acides aminés, et qui est reconnue par au moins un anticorps anti-C33. Ainsi, on entend par "séquence dérivée", notamment, les séquences dans lesquelles un ou plusieurs acides aminés est remplacé par un ou plusieurs autres acides aminés, comme illustré à l'Exemple 5 ci-après ; les séquences dans lesquelles un ou plusieurs acide aminé de la série L est remplacé par un acide aminé de la série D, et vice-versa ; les séquences dans lesquelles on a introduit une modification des chaînes latérales des acides aminés, telle que par exemple une acétylation des fonctions amines, une carboxylation des fonctions thiols, une estérification des fonctions carboxyliques ; une modification des liaisons peptidiques telles que par exemple des liaisons carba, retro, inverso, retro-inverso, réduites et méthylène-oxy (voir notamment PCT WO 94/05 311),

Par "sera d'individus ou d'animaux infectés par VHC," on entend ici des sera de patients ayant contracté une infection à VHC et qui contiennent des immunoglobulines reconnaissant spécifiquement au moins l'antigène C33 du virus VHC.

Par "polynucléotide", on entend soit une séquence d'ADN, soit une séquence d'ARN, soit une séquence d'ADNc résultant de la transcription inverse d'une séquence d'ARN, d'origine naturelle ou de synthèse, avec ou sans bases modifiées.

La présente invention a pour objet un polypeptide capable de réagir spécifiquement avec un anticorps anti-C33, et dont la séquence comprend tout ou partie de SEQ ID N01, ou une séquence dérivée. Les peptides de l'invention contiennent au moins 5, et en particulier au moins 6 acides aminés.

La séquence du peptide de l'invention comprend tout ou partie des séquences SEQ ID N02, SEQ ID N03, SEQ ID N04, SEQ ID N05, SEQ ID N06, SEQ ID N07 et SEQ ID N08, et en particulier la séquence dudit fragment correspond à SEQ ID N010, ou encore des séquences dérivées correspondantes telles que SEQ ID N09, et notamment les séquences SEQ ID N032 à SEQ ID N037.

Ainsi, l'invention concerne notamment des séquences peptidiques choisies parmi les séquences d'au moins 5 (et en particulier d'au moins 6) acides aminés consécutifs présents dans les séquences indiquées.

Ces polypeptides peuvent être préparés selon les méthodes connues. Par ailleurs, les polypeptides de l'invention peuvent être conjugués selon des techniques bien connues de l'homme du métier, à une molécule porteuse (comme par exemple une protéine naturelle ou recombinante autre qu'un antigène naturel ou recombinant de VHC, un polymère synthétique d'acides aminés ou de chaînes aliphatiques, un fragment nucléique) ou à une molécule traceuse (comme par exemple un oligonucléotide, une enzyme telle que notamment la peroxydase de raifort, une phosphatase alcaline ou une galactosidase) ou encore à un radioélément. Les peptides de l'invention peuvent aussi être fixés sur un support. La molécule porteuse ne doit évidemment pas réagir avec les anticorps anti-C33.

Un second objet de la présente invention est une application d'un polypeptide décrit ci-dessus et consiste en un réactif pour la détection d'une infection par VHC, ledit réactif comprenant, à titre de substance réactive, au moins un polypeptide de l'invention.

Un troisième objet de l'invention est un kit de détection d'une infection par le VHC, comprenant le réactif décrit ci-dessus, fixé sur un support solide, immunologiquement compatible avec ledit réactif.

Le terme support solide tel qu'utilisé ici est sans limitation sous la forme d'une plaque de microtitration, d'une feuille, d'un cône, d'un puits, d'une bille, ou tout substrat micro-particulaire approprié, et inclut tous les matériaux sur lesquels peuvent être immobilisés les fragments peptidiques de l'invention. Ce support peut être réalisé en tous matériaux appropriés, notamment des polysaccharides, tels que les matériaux de cellulose, par exemple du papier, des dérivés de cellulose tels que la nitrocellulose et l'acétate de cellulose ; des polymères tels que chlorure de vinyle, polyéthylène, polystyrène, polyacrylate, ou des copolymères tels que les copolymères de chlorure de vinyle et de propylène, les copolymères de chlorure de vinyle et d'acétate de vinyle; les copolymères à base de styrène; des fibres naturelles telles que le coton et des fibres synthétiques telles que le nylon. De préférence, le support solide est un polymère de polystyrène, un copolymère butadiène-styrène ou un copolymère butadiène-styrène en mélange avec un ou plusieurs polymères ou copolymères choisis parmi le polystyrène, les copolymères styrène-acrylonitrile ou styrène-méthacrylate de méthyle, les polypropylènes, les polycarbonates ou analogues.

La fixation du réactif sur le support solide peut être réalisée de manière directe ou indirecte.

De manière directe, deux approches sont possibles : soit par adsorption du réactif sur le support solide, c'est-à-dire par des liaisons non covalentes (principalement de type hydrogène, Van der Wals ou ionique), soit par établissement de liaisons covalentes entre le réactif et le support. De manière indirecte, on peut fixer préalablement (par adsorption ou covalence) sur le support solide un composé "anti-réactif' capable d'interagir avec le réactif de façon à immobiliser l'ensemble sur le support solide.

L'invention concerne en outre un procédé de détection et/ou de séparation et/ou de purification et/ou de dosage d'anticorps anti-C33 éventuellement présents dans un échantillon, dans lequel on utilise un peptide selon l'invention pour former un complexe immun avec lesdits anticorps, s'ils sont présents dans l'échantillon.

L'invention concerne notamment un procédé pour la détection et/ou la concentration, et/ou la séparation, et/ou la purification, et/ou le dosage d'anticorps anti-C33, dans un échantillon de fluide biologique comprenant les étapes suivantes : on met en contact ledit échantillon avec un réactif de l'invention, dans des conditions permettant une réaction immunologique, puis on détecte, sépare et/ou quantifie le complexe immun éventuellement formé.

L'invention concerne également un procédé pour le dosage de l'antigène C33 de VHC dans un échantillon d'un fluide biologique effectué par une technique de compétition : on met simultanément en contact ledit échantillon avec une quantité prédéterminée d'anticorps anti-C33 et avec une quantité prédéterminée d'un réactif de l'invention, et on détermine la quantité d'antigène C33 dans ledit échantillon par déduction à partir de la quantité mesurée du complexe formé entre le réactif et lesdits anticorps anti-C33.

L'invention concerne également un procédé de l'invention pour le dosage de l'antigène C33 dans un échantillon d'un fluide biologique, selon lequel dans un premier temps, on met en contact ledit échantillon avec une quantité prédéterminée d'anticorps anti-C33, dans un second temps, on ajoute une quantité prédéterminée d'un réactif de l'invention, et on détermine la quantité d'antigène C33 dans ledit échantillon par déduction à partir de la quantité mesurée du complexe formé entre ledit réactif et lesdits anticorps anti-C33.

Ces procédés sont mis en oeuvre de façon connue. Ils peuvent être basés sur une méthode radioimmunologique de type RIA, RIPA (essai par précipitation radioimmunologique) ou IRMA (essai radioimmunométrique) ou une méthode immunoenzymatique de type Western-blot ou ELISA.

Une autre application des polypeptides de l'invention est une composition immunothérapeutique active, notamment une préparation vaccinale, comprenant, à titre de principe actif un polypeptide décrit ci-dessus, ledit principe actif étant éventuellement sous forme de conjugué, et éventuellement un excipient pharmaceutiquement acceptable. La formation de conjugués est bien connue et ne sera pas rappelée ici.

L'invention apporte en outre un polynucléotide codant un polypeptide décrit ci-dessus. La séquence d'un tel polynucléotide peut évidemment varier, compte tenu de la dégénérescence du code génétique. Ce polynucléotide comprend par exemple une séquence nucléotidique telle que la séquence SEQ ID N011 ou des fragments de celle-ci.

Elle concerne aussi une cassette d'expression fonctionnelle permettant l'expression d'un polynucléotide de l'invention et comprenant ce dernier, ainsi qu'un vecteur dans lequel est intégrée ladite cassette d'expression, et un système cellulaire eucaryote ou procaryote comprenant une cassette d'expression ou un vecteur tels que présentés ci-dessus.

### Exemple 1 : Sélection d'un clone phagique codant un hexapeptide capable de réagir spécifiquement avec un anticorps monoclonal anti-C33.

Le criblage d'une banque d'expression d'hexapeptides, construite dans le phage M13 selon la méthode décrite par Scott et Smith (1990, Science, 249, 386-390), par un anticorps monoclonal spécifique de l'antigène C33 de la région NS3 du virus de l'hépatite C (anticorps monoclonal anti-C33 noté 70-13, Réf. M040018Y, commercialisé par Anogen) a permis d'identifier un hexapeptide de séquence peptidique Lys-Phe-Ser-Ser-Arg-Leu. La réponse immunologique du clone phagique portant cet hexapeptide a été testée en ELISA vis-à-vis, d'une part de l'anticorps ayant permis la sélection, et d'autre part de onze autres anticorps monoclonaux anti-C33 obtenus par la demanderesse et d'un anticorps monoclonal témoin E1E7 qui est spécifique de l'antigène P30 de *Toxoplasma gondii.* Les tests ELISA sont réalisés selon le schéma suivant : 100 µl d'anticorps anti-phages M13 (commercialisé par Prim, Inc. Boulder, CO 80303) dilués au 1/500 dans une solution NaHCO₃ 0,1M pH 8,6 sont fixés dans les puits de plaques de microtitration Nunc maxisorb (nom commercial). Après deux lavages avec du TBS/Tween 0,05 %, 100 µl de préparation phagique purifiée sont ensuite incubés pendant deux heures à 37°C dans les puits afin d'obtenir la fixation des phages dans les puits. La saturation des sites spécifiques est réalisée à l'aide de TBS contenant 1 % de BSA pendant deux heures à 37°C. Après trois lavages avec du TBS(Tris 50mM pH 7,5 150mM NaCl)/Tween 0,05 %, 100 µl de l'anticorps monoclonal anti-C33 (Anogen) sont ajoutés et incubés une nuit à 4°C.

Après quatre lavages au TBS/Tween 0,05 %, 100 µl de conjugué anti-souris marqué à la peroxydase (commercialisé par Jackson Immuno Research Laboratories Inc.) sont ajoutés, et l'ensemble est incubé pendant une heure à 37°C. La réaction enzymatique de révélation est effectuée en ajoutant 100 µl d'une solution H₂O₂/ortho-phénylène-diamine (OPD) et en incubant l'échantillon 30 minutes à température ambiante. La réaction de coloration est interrompue par l'addition de 50 µl d'acide sulfurique 1,8N. La densité optique est mesurée sur un lecteur de plaques Bio Mérieux à 492nm.

Les résultats montrent que plusieurs anticorps monoclonaux testés reconnaissent le clone phagique sélectionné portant l'hexapeptide Lys-Phe-Ser-Ser-Arg-Leu (SEQ ID N010). La réponse témoin avec l'anticorps anti-P30 est négative. Par ailleurs, il a été vérifié, pour chacun des anticorps monoclonaux testés, qu'aucune réaction n'était observée entre lesdits anticorps et un clone phagique dans lequel aucun peptide n'a été cloné.

### Exemple 2 : Inhibition par la protéine C33 recombinante de la réponse ELISA obtenue avec le clone phagique identifié

Un test d'inhibition en ELISA a été effectué, afin de déterminer si la réponse obtenue avec le clone phagique identifié est inhibée par une pré-incubation de l'anticorps monoclonal anti-C33 70-13 avec une protéine C33 recombinante.

La protéine C33 recombinante utilisée dans cet exemple a été obtenue par amplification spécifique (PCR) de l'ARN correspondant à la région NS3 du virus de l'hépatite C à partir d'un sérum de patient atteint de l'hépatite C (génotype 1a). Le gène NS3 ainsi amplifié a été cloné dans le vecteur d'expression pMH [Cheynet V. et al., (1993) Protein Expression and Purification, 4, 367-372]. La protéine C33 est exprimée sous la forme d'une protéine de fusion correspondant à la séquence 1192-1457 déduite en acides aminés du génome VHC (Choo, Q.L., (1989) Science, 244, 359-362) assortie de 6 histidines en position N-terminale. La protéine C33 recombinante synthétisée a été purifiée par chromatographie d'affinité sur colonne métal-chélate (Résine Ni-NTA, Quiagen).

Le test ELISA est réalisé comme décrit dans l'exemple 1 avec les modifications suivantes : l'anticorps monoclonal 70-13 (20nM) est pré-incubé avec différentes concentrations de protéine C33 recombinante pendant 45 minutes à 37°C, puis le mélange est ajouté dans les puits de la plaque ELISA dans lesquels sont fixés les phages correspondant à l'hexapeptide Lys-Phe-Ser-Ser-Arg-Leu. La plaque est incubée pendant 2 heures à 37°C.

Les résultats obtenus montrent que la réponse en ELISA est inhibée de 33 % pour une concentration en protéine C33 recombinante de 30 µg/ml. Cette diminution de la réponse immunoenzymatique met clairement en évidence que le clone phagique identifié est reconnu par les mêmes anticorps capables de reconnaître la protéine recombinante C33.

### Exemple 3 : Spécificité des anticorps humains anti-VHC pour le clone phagique identifié portant la séquence Lys-Phe-Ser-Ser-Arg-Leu (SEQ ID N010)

Plusieurs des anticorps monoclonaux testés reconnaissent un site immunodominant sur la protéine C33 et sur le clone phagique renfermant l'hexapeptide Lys-Phe-Ser-Ser-Arg-Leu. On a cherché à savoir si ce même clone phagique est également reconnu par les anticorps humains présents dans le sérum de patients infectés par le virus de l'hépatite C.

Le test ELISA est réalisé dans des conditions de compétition en deux étapes : dans un premier temps, le clone phagique à tester est mis en contact avec une fraction d'anticorps humains anti-VHC, et dans un second temps avec un anticorps monoclonal dont on connaît la réactivité vis-à-vis dudit clone. Des tests sont réalisés avec différentes dilutions de la fraction d'anticorps humains anti-VHC et avec une quantité constante d'anticorps monoclonal. Si les anticorps humains sont capables de reconnaître le clone phagique testé, une inhibition de la fixation de l'anticorps monoclonal sur ledit clone doit être observée pour des facteurs de dilutions décroissants.

La fraction "anticorps humains" est constituée par un mélange de 5 sérums de patients infectés par VHC pour lesquels la présence d'anticorps anti-C33 a été vérifiée à l'aide de protéine C33 recombinante. L'anticorps monoclonal 12A1H2 (Bio Mérieux) est choisi pour la seconde étape car il s'agit de l'anticorps pour lequel on observe la plus forte réactivité vis-à-vis du clone phagique étudié dans les réactions conduites dans l'exemple 1.

100 µl de différentes dilutions dudit mélange de sérums sont mis en contact avec le clone phagique identifié, immobilisé comme décrit dans l'exemple 1, pendant 45 minutes à 37°C. Après deux lavages au TBS/Tween 0,05 %, 100 µl d'anticorps monoclonal 12A1H2 à la concentration de 50nM sont ajoutés et incubés durant une nuit à 4°C. Après quatre lavages au TBS/Tween, 100 µl de conjugué anti-souris marqué à la peroxydase sont ajoutés et incubés pendant une heure à 37°C. La réaction de révélation est effectuée en ajoutant 100 µl d'une solution H₂O₂/ortho-phénylène-diamine (OPD) et en incubant l'échantillon 30 minutes à température ambiante. La réaction de coloration est interrompue par l'addition de 50 µl d'acide sulfurique 1,8N. La densité optique est mesurée sur un lecteur de plaque Bio Mérieux à 492nm. Par ailleurs, un contrôle a été réalisé, dans les mêmes conditions réactionnelles mais avec un mélange de sérums humains non infectés par VHC (sérums négatifs) afin de vérifier la spécificité de la réaction éventuelle entre le clone phagique testé et le mélange de sérums humains hépatite C(+). De même, un contrôle a été effectué, dans lequel la réactivité du mélange de sérums séropositifs a été testée, dans les mêmes conditions expérimentales, à l'égard du phage M13 non modifié.

Les résultats indiquent que pour une dilution au 1/100 du mélange de sérums humains, on observe une inhibition de la fixation de l'anticorps monoclonal 12A1H2 de 40 %, et pour une dilution au 1/10, l'inhibition est d'environ 60 %. Cet exemple montre donc que le clone phagique renfermant la séquence Lys-Phe-Ser-Ser-Arg-Leu est bien reconnu par des anticorps présents dans les sera des malades infectés par VHC, et que ces anticorps entrent en compétition avec l'anticorps monoclonal anti-C33 12A1H2 pour leur fixation audit clone phagique.

### Exemple 4 : Synthèses multiples d'hexapeptides chevauchants et analyse immunologique desdits peptides

Les exemples précédents exposent des études conduites sur un hexapeptide donné, cloné dans une structure phagique plus complexe. On a également recherché si cette même séquence peptidique, en tant que telle, pouvait conserver ses propriétés immunologiques à l'égard notamment des anticorps monoclonaux 70-13 (commercialisé par Anogen) et 12A1H12 (Bio Mérieux), et plus particulièrement à l'égard du mélange de sérums humains utilisé dans l'exemple 3.

Pour cette étude, et afin de déterminer le rôle éventuel des acides aminés propres à la séquence du phage M13, qui sont contigus à la séquence de l'hexapeptide identifié dans le clone isolé de la banque d'expression (voir exemple 1), une série d'octapeptides chevauchants couvrant la séquence Tyr-Ser-His-Ser-Ala-Asp-Gly-Ala-[Lys-Phe-Ser-Ser-Arg-Leu]-Gly-Ala-Ala-Gly-Ala-Glu-Thr-Val-Glu-Ser-Cys-Leu (SEQ ID N012) a été synthétisée.

Les octapeptides chevauchants synthétisés et analysés selon la technique Spotscan sont, dans l'ordre :

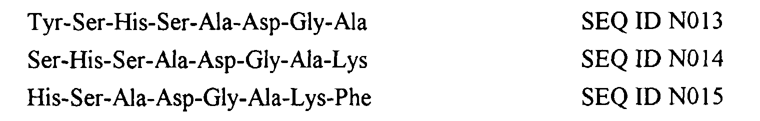

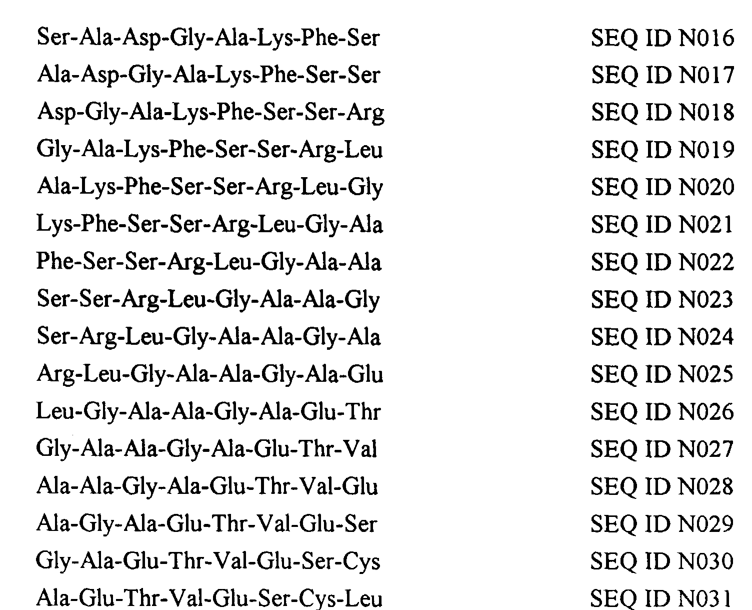

La synthèse a été réalisée sur membrane de cellulose activée suivant la technique développée par Berg et al. (1989, J. Ann. Chem. Soc., 111, 8024-8026) et commercialisée par Cambridge Research Biochemicals (nom commercial : Spotscan). Cette technique permet la synthèse simultanée d'un grand nombre de peptides. Les acides aminés estérifiés utilisés lors de la synthèse présentent un groupement α-aminé protégé par un groupement FMOC (Nova Biochem) et des groupements latéraux protégés par des groupements protecteurs tels que, par exemple, trityle, t-butyl ester ou t-butyl éther. Les acides aminés estérifiés sont solubilisés dans la N-méthyl pyrrolidone (NMP) à la concentration de 300nM et 0,9 µl sont déposés au niveau de taches de dépôt de bleu de bromophénol. Après une incubation de 15 minutes, un nouveau dépôt d'acides aminés est réalisé suivi d'une autre incubation de 15 minutes. Si le couplage entre les deux acides aminés s'est effectué correctement, on observe un changement de coloration (passage du bleu au vert-jaune). Après trois lavages dans du DMF, une étape d'acétylation est réalisée par de l'anhydride acétique. Puis les groupements aminés terminaux des peptides en cours de synthèse sont déprotégés par de la pipéridine en solution à 20 % dans le DMF. Les taches de dépôt sont recolorées par une solution de bleu de bromophénol à 1 % dans le DMF, lavées trois fois au méthanol et séchées. L'ensemble de ces opérations constitue un cycle d'addition d'un acide aminé et ce cycle est répété jusqu'à la fin de la synthèse. Lorsque tous les acides aminés ont été ajoutés, le groupement NH₂-terminal du dernier acide aminé est déprotégé par de la pipéridine en solution à 20 % dans le DMF et acétylé par de l'anhydride acétique. Les groupements protecteurs de la chaîne latérale sont enlevés par un mélange dichlorométhane: acide trifluoroacétique:triisobutylsilane (5ml: 5ml:250 µl). L'immunoréactivité des peptides ainsi synthétisés peut alors être testée par ELISA.

Après synthèse des différents octapeptides, la membrane de cellulose est rincée avec du méthanol, lavée dans du TBS (Tris 0,1M pH7,2) puis incubée pendant une nuit à température ambiante dans du tampon de saturation (commercialisé par Cambridge Research Biochemicals). Après plusieurs lavages avec du TBS-T (Tris 0,1M pH7,2 - 0,05 % Tween 20), la membrane est mise en contact d'une solution d'anticorps monoclonaux anti-C33 70-13 ou 12A1H2 (100nM), et incubée 4 heures à température ambiante. Après trois lavages avec du TBS-T, le conjugué anti-immunoglobulines de souris marqué à la β-galactosidase (commercialisé par Cambridge Research Biochemicals) est ajouté à une dilution au 1/200 et l'ensemble est incubé deux heures à température ambiante. Après plusieurs lavages de la membrane par du TBS-Tween 0,05 % et du PBS, l'immunoréactivité au niveau des différentes taches est révélée par l'addition d'une solution de substrat (5-bromo-4-chloro-3-indoyl-β-D-galactopyranoside dans du PBS contenant du chlorure de magnésium et du ferricyanure de potassium) et incubation pendant 10 à 40 minutes. La coloration des taches est estimée qualitativement sur une échelle numérique allant de O à 5.

L'analyse séquentielle des résultats obtenus montre que l'on obtient une première coloration avec la séquence Asp-Gly-Ala-Lys-Phe-Ser-Ser-Arg. Puis, la coloration bleue augmente progressivement lorsqu'on avance le long de la séquence Tyr-Ser-His-Ser-Ala-Asp-Gly-Ala-[Lys-Phe-Ser-Ser-Arg-Leu]-Gly-Ala-Ala-Gly-Ala-Glu-Thr-Val-Glu-Ser-Cys-Leu, pour atteindre un maximum avec les séquences Lys-Phe-Ser-Ser-Arg-Leu-Gly-Ala et Phe-Ser-Ser-Arg-Leu-Gly-Ala-Ala. Une très légère coloration est encore visible pour la séquence Ser-Arg-Leu-Gly-Ala-Ala-Gly-Ala qui disparaît complètement à partir de la séquence Arg-Leu-Gly-Ala-Ala-Gly-Ala-Glu.

L'immunoréactivité des différents peptides synthétisés chimiquement sur membrane de cellulose a ensuite été testée avec le mélange de sérums humains utilisé dans l'exemple 3. Les résultats obtenus avec une dilution de ce mélange au 1/50 et une dilution au 1/200 du conjugué anti-immunoglobulines humaines marqué à la β-galactosidase (commercialisé par Cambridge Research Biochemicals) montre une coloration maximale pour le peptide Phe-Ser-Ser-Arg-Leu-Gly-Ala-Ala.

### Exemple 5 : Substitutions d'acides aminés dans le peptide Lys-Phe-Ser-Ser-Arg-Leu-Gly-Ala et analyse immunologique desdits peptides modifiés

Afin de déterminer dans quelle mesure la nature des différents acides aminés constituant le peptide Lys-Phe-Ser-Ser-Arg-Leu-Gly-Ala peut varier sans réduire les propriétés antigéniques dudit peptide l'égard des anticorps anti-C33, des essais de substitutions ponctuelles ont été effectués.

Les peptides ont été synthétisés selon la méthode décrite dans l'exemple précédent, dite "Spotscan", en substituant sur le peptide chaque acide aminé à une position donnée par les dix-neuf autres. Ces peptides ont ensuite été testés tel que décrit précédemment. L'importance de chaque résidu peut ainsi être examinée et un peptide regroupant les meilleurs acides aminés à chaque position peut être ensuite synthétisé.

Les résultats obtenus montrent que :
- lorsque l'Arginine en position 5 est remplacée par la Glycine ou la Proline ou la Tyrosine, on observe une meilleure réponse,
- la Phénylalanine en position 2 peut être remplacée par la Tyrosine : on observe alors une réponse équivalente,
- dans le cas d'une double substitution, la Phénylalanine en position 2 peut avantageusement être remplacée par l'Alanine ou 1'Histidine si l'acide aminé en position 5 est une Tyrosine.

Ces résultats sont donc obtenus avec les séquences suivantes :

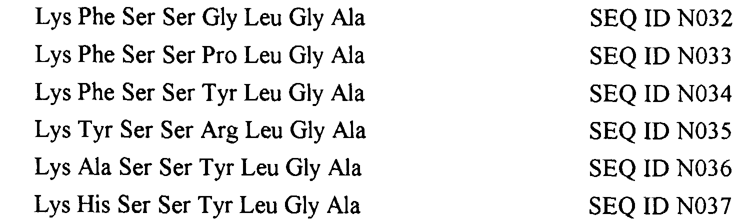

## Revendications

1. Polypeptide capable de réagir spécifiquement avec un anticorps dirigé contre l'antigène C33 du virus de l'hépatite C, caractérisé en ce que sa séquence peptidique, qui comporte au moins 5 acides aminés, comprend tout ou partie de SEQ ID N01, ou une séquence dérivée de ladite séquence peptidique.

2. Polypeptide selon la revendication 1, caractérisé en ce que sa séquence peptidique comprend une séquence choisie parmi SEQ ID N02, SEQ ID N03, SEQ ID N04, SEQ ID N05, SEQ ID N06, SEQ ID N07, SEQ ID N08 et SEQ ID N10, ou une séquence dérivée desdites séquences.

3. Polypeptide selon la revendication 2, caractérisé en ce que ladite séquence dérivée est choisie parmi les séquences représentées par SEQ ID N09, et en particulier les séquences SEQ ID N032 à 37.

4. Polypeptide selon la revendication 1, caractérisé en ce que sa séquence peptidique consiste en tout ou partie de la séquence peptidique SEQ ID N01, ou une séquence dérivée.

5. Polypeptide selon la revendication 4, caractérisé en ce que sa séquence peptidique consiste en une séquence peptidique choisie parmi SEQ ID N02 à 10 et 32 à 37.

6. Réactif pour la détection d'une infection par VHC, caractérisé en ce qu'il comprend, à titre de substance réactive, au moins un polypeptide selon l'une quelconque des revendications 1 à 5, ledit polypeptide étant éventuellement marqué.

7. Kit de détection d'une infection par VFIC, caractérisé en ce qu'il comprend un réactif selon la revendication 6 fixé sur un support solide, immunologiquement compatible avec ledit réactif.

8. Procédé de détection, de séparation, de purification et/ou de dosage de l'antigène C33 ou d'anticorps anti-C33, caractérisé en ce que l'on utilise comme réactif un peptide tel que défini dans l'une des revendications 1 à 5.

9. Procédé selon la revendication 8, pour la détection, la séparation, la purification, et/ou le dosage d'anticorps anti-C33, dans un échantillon de fluide biologique, caractérisé en ce qu'on utilise un peptide tel que défini dans l'une quelconque des revendication 1 à 5, pour former un complexe immun avec lesdits anticorps, s'ils sont présents, et en ce qu'on détecte, sépare et/ou quantifie le complexe immun éventuellement formé.

10. Procédé selon la revendication 8, pour le dosage de l'antigène C33 dans un échantillon d'un fluide biologique, par une technique de compétition, caractérisé en ce qu'on met simultanément en contact ledit échantillon avec une quantité prédéterminée d'anticorps anti-C33, et une quantité prédéterminée d'un réactif selon la revendication 6, et on détermine la quantité d'antigène C33 dans ledit échantillon par déduction à partir de la quantité mesurée du complexe immun formé entre le réactif et lesdits anticorps anti-C33.

11. Procédé selon la revendication 8, pour le dosage de l'antigène C33 dans un échantillon d'un fluide biologique, caractérisé en ce que dans un premier temps, on met en contact ledit échantillon avec une quantité prédéterminée d'anticorps anti-C33, dans un second temps, on ajoute une quantité prédéterminée d'un réactif selon la revendication 6, et on détermine la quantité d'antigène C33 dans ledit échantillon par déduction à partir de la quantité mesurée du complexe immun formé entre le réactif et lesdits anticorps anti-C33.

12. Composition immunothérapeutique active, notamment préparation vaccinale, caractérisée en ce qu'elle comprend à titre de principe actif un polypeptide selon l'une quelconque des revendications 1 à 5, ledit principe actif étant éventuellement sous forme de conjugué, et éventuellement un excipient pharmaceutiquement acceptable.

13. Polynucléotide comprenant une séquence nucléotidique codant un polypeptide selon l'une quelconque des revendications 1 à 5.

14. Cassette d'expression fonctionnelle permettant l'expression d'un polynucléotide selon la revendication 13.

15. Vecteur comprenant une cassette d'expression selon la revendication 14.

16. Matériel cellulaire eucaryote ou procaryote comprenant une cassette d'expression selon la revendication 14 ou un vecteur selon la revendication 15.


LISTE DE SEQUENCES

(1) INFORMATIONS GENERALES :

(i) DEPOSANT :
(A) NOM : BIO MERIEUX
(B) RUE : Chemin de l'Orme
(C) VILLE : MARCY L'ETOILE
(E) PAYS : FRANCE
(F) CODE POSTAL : 69280
(G) TELEPHONE : (33) 78 87 20 00
(H) TELECOPIE : (33) 78 87 20 90

(ii) TITRE DE L' INVENTION : Peptide capable d'être reconnu par des anticorps
reconnaissant l'antigène C33 du virus de l'hépatite C

(iii) NOMBRE DE SEQUENCES : 37

(iv) FORME DECHIFFRABLE PAR ORDINATEUR :
(A) TYPE DE SUPPORT : Disquette
(B) ORDINATEUR : PC compatible
(C) SYSTEME D' EXPLOITATION : MS-DOS Version #6
(D) LOGICIEL : WORD Version #6 POUR WINDOWS #3.1

(vi) DONNEES DE LA DEMANDE ANTERIEURE :
(A) NUMERO DE LA DEMANDE : FR 95 09005
(B) DATE DE DEPOT : 25-JUL-1995

(2) INFORMATIONS POUR LA SEQ ID N0 : 1 :

(i) CARACTERISTIQUES DE LA SEQUENCE :
(A) LONGUEUR : 14 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 1 :

Asp Gly Ala Lys Phe Ser Ser Arg Leu Gly Ala Ala Gly Ala
1               5                   10

(2) INFORMATIONS POUR LA SEQ ID N0 : 2 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 2 :

Asp Gly Ala Lys Phe Ser Ser Arg
1               5

(2) INFORMATIONS POUR LA SEQ ID N0 : 3 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 3 :

Gly Ala Lys Phe Ser Ser Arg Leu
1               5

(2) INFORMATIONS POUR LA SEQ ID N0 : 4 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 4 :

Ala Lys Phe Ser Ser Arg Leu Gly
1               5

(2) INFORMATIONS POUR LA SEQ ID N0 : 5 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 5 :

Lys Phe Ser Ser Arg Leu Gly Ala
1               5

(2) INFORMATIONS POUR LA SEQ ID N0 : 6 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 6 :

Phe Ser Ser Arg Leu Gly Ala Ala
1               5

(2) INFORMATIONS POUR LA SEQ ID N0 : 7 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 7 :

Ser Ser Arg Leu Gly Ala Ala Gly
1               5

(2) INFORMATIONS POUR LA SEQ ID N0 : 8 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 8 :

Ser Arg Leu Gly Ala Ala Gly Ala
1               5

(2) INFORMATIONS POUR LA SEQ ID N0 : 9 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(ix) CARACTERISTIQUE:
(A) NOM/CLE: Peptide
(B) EMPLACEMENT:2..5
(D) AUTRES INFORMATIONS:/note = Xaa en position 2 = Tyr ou Phe ou
Ala ou His
Xaa en position 5 = Arg ou Gly ou
Pro ou Tyr

(xi) DESCRIPTION DE LA SEQUENCE : SEQ ID N0 : 9 :

Lys Xaa Ser Ser Xaa Leu Gly Ala
1               5

(2) INFORMATIONS POUR LA SEQ ID N0 : 10 :

(i) CARACTERISTIQUES DE LA SEQUENCE :
(A) LONGUEUR : 6 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 10 :

Lys Phe Ser Ser Arg Leu
1               5

(2) INFORMATIONS POUR LA SEQ ID N0 : 11 :

(i) CARACTERISTIQUES DE LA SEQUENCE :
(A) LONGUEUR : 42

(B) TYPE : nucléotide
(C) NOMBRE DE BRINS: simple
(D) CONFIGURATION : linéaire

(ii) TYPE DE MOLECULE : ADN

(xi) DESCRIPTION DE LA SEQUENCE : SEQ ID N0 : 11 :

GAYGGNGCNA ARTTYTCNTC NCGNCTNGGN GCNGCNGGNG CN 42

(2) INFORMATIONS POUR LA SEQ ID N0 : 12 :

(i) CARACTERISTIQUES DE LA SEQUENCE :
(A) LONGUEUR : 26 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 12 :

Tyr Ser His Ser Ala Asp Gly Ala Lys Phe Ser Ser Arg Leu Gly Ala
1               5                   10                  15

Ala Gly Ala Glu Thr Val Glu Ser Cys Leu
            20                  25

(2) INFORMATIONS POUR LA SEQ ID N0 : 13 :

(i) CARACTERISTIQUES DE LA SEQUENCE :
(A) LONGUEUR : 8 acides aminés

(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 13 :

Tyr Ser His Ser Ala Asp Gly Ala
1               5

(2) INFORMATIONS POUR LA SEQ ID N0 : 14 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 14 :

Ser His Ser Ala Asp Gly Ala Lys
1               5

(2) INFORMATIONS POUR LA SEQ ID N0 : 15 :

(i) CARACTERISTIQUES DE LA SEQUENCE :
(A) LONGUEUR : 8 acides aminés
(B) TYPE : acide aminé

(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 15 :

His Ser Ala Asp Gly Ala Lys Phe
1               5

(2) INFORMATIONS POUR LA SEQ ID N0 : 16 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 16 :

Ser Ala Asp Gly Ala Lys Phe Ser
1               5

(2) INFORMATIONS POUR LA SEQ ID N0 : 17 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 17 :

Ala Asp Gly Ala Lys Phe Ser Ser
1               5

(2) INFORMATIONS POUR LA SEQ ID N0 : 18 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 18 :

Asp Gly Ala Lys Phe Ser Ser Arg
1               5

(2) INFORMATIONS POUR LA SEQ ID N0 : 19 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 19 :

Gly Ala Lys Phe Ser Ser Arg Leu
1               5

(2) INFORMATIONS POUR LA SEQ ID N0 : 20 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 20 :

Ala Lys Phe Ser Ser Arg Leu Gly
1               5

(2) INFORMATIONS POUR LA SEQ ID N0 : 21 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 21 :

Lys Phe Ser Ser Arg Leu Gly Ala
1               5

(2) INFORMATIONS POUR LA SEQ ID N0 : 22 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 22 :

Phe Ser Ser Arg Leu Gly Ala Ala
1               5

(2) INFORMATIONS POUR LA SEQ ID N0 : 23 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 23 :

Ser Ser Arg Leu Gly Ala Ala Gly
1               5

(2) INFORMATIONS POUR LA SEQ ID N0 : 24 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 24 :

Ser Arg Leu Gly Ala Ala Gly Ala
1               5

(2) INFORMATIONS POUR LA SEQ ID N0 : 25 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 25 :

Arg Leu Gly Ala Ala Gly Ala Glu
1               5

(2) INFORMATIONS POUR LA SEQ ID N0 : 26 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 26 :

Leu Gly Ala Ala Gly Ala Glu Thr
1               5

(2) INFORMATIONS POUR LA SEQ ID N0 : 27 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 27 :

Gly Ala Ala Gly Ala Glu Thr Val
1               5

(2) INFORMATIONS POUR LA SEQ ID N0 : 28 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 28 :

Ala Ala Gly Ala Glu Thr Val Glu
1               5

(2) INFORMATIONS POUR LA SEQ ID N0 : 29 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 29 :

Ala Gly Ala Glu Thr Val Glu Ser
1               5

(2) INFORMATIONS POUR LA SEQ ID N0 : 30 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 30 :

Gly Ala Glu Thr Val Glu Ser Cys
1               5

(2) INFORMATIONS POUR LA SEQ ID N0 : 31 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 31 :

Ala Glu Thr Val Glu Ser Cys Leu
1               5

(2) INFORMATIONS POUR LA SEQ ID N0 : 32 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 32 :

Lys Phe Ser Ser Gly Leu Gly Ala
1               5

(2) INFORMATIONS POUR LA SEQ ID N0 : 33 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 33 :

Lys Phe Ser Ser Pro Leu Gly Ala
1               5

(2) INFORMATIONS POUR LA SEQ ID N0 : 34 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 34 :

Lys Phe Ser Ser Tyr Leu Gly Ala
1               5

(2) INFORMATIONS POUR LA SEQ ID N0 : 35 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 35 :

Lys Tyr Ser Ser Arg Leu Gly Ala
1               5

(2) INFORMATIONS POUR LA SEQ ID N0 : 36 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 36 :

Lys Ala Ser Ser Tyr Leu Gly Ala
1               5

(2) INFORMATIONS POUR LA SEQ ID N0 : 37 :

(i) CARACTERISTIQUES DE LA SEQUENCE :
(A) LONGUEUR : 8 acides aminés
(B) TYPE : acide aminé
(D) CONFIGURATION : non connue

(ii) TYPE DE MOLECULE : peptide

(v) TYPE DE FRAGMENT : N-terminal ou C-terminal ou interne

(xi) DESCRIPTION DE LA SEQUENCE : SEQ ID N0 : 37 :

Lys His Ser Ser Tyr Leu Gly Ala
1               5